(19) Europäisches Patentamt
European Patent Office
Office européen des brevets

(11) Veröffentlichungsnummer: **0 683 169 A1**

# (12) EUROPÄISCHE PATENTANMELDUNG

(21) Anmeldenummer: **95106401.3**

(22) Anmeldetag: **28.04.95**

(51) Int. Cl.[6]: **C07D 498/06**, A61K 31/535, //(C07D498/06,273:00,221:00)

(30) Priorität: **11.05.94 DE 4416620**

(43) Veröffentlichungstag der Anmeldung: **22.11.95 Patentblatt 95/47**

(84) Benannte Vertragsstaaten: **AT BE CH DE DK ES FR GB GR IE IT LI NL PT SE**

(71) Anmelder: **BAYER AG**

**D-51368 Leverkusen (DE)**

(72) Erfinder: **Jaetsch, Thomas, Dr.**
**Eintrachtstrasse 105**
**D-50668 Köln (DE)**
Erfinder: **Petersen, Uwe, Dr.**
**Auf dem Forst 4**
**D-51375 Leverkusen (DE)**
Erfinder: **Bremm, Klaus-Dieter, Dr.**
**Eberhardstrasse 20**
**D-45661 Recklinghausen (DE)**
Erfinder: **Endermann, Rainer, Dr.**
**In den Birken 152A**
**D-42113 Wuppertal (DE)**
Erfinder: **Metzger, Karl-Georg, Dr.**
**Pahlkestrasse 75**
**D-42115 Wuppertal (DE)**
Erfinder: **Scheer, Martin, Dr.**
**Herberts Katernberg 7**
**D-42113 Wuppertal (DE)**
Erfinder: **Stegemann, Michael, Dr.**
**Grundermühlenweg 29**
**D-51381 Leverkusen (DE)**
Erfinder: **Wetzstein, Heinz-Georg, Dr.**
**Schleiermacherstrasse 13**
**D-51377 Leverkusen (DE)**

(54) **8-Amino-pyrido [1,2,3-d,e] [1,3,4]benzoxadiazinderivate sowie diese enthaltende antibakterielle Mittel.**

(57) Die vorliegende Erfindung betrifft neue 8-Amino-pyrido[1,2,3-d,e][1,3,4]benzoxadiazinderivate der allgemeinen Formel (I)

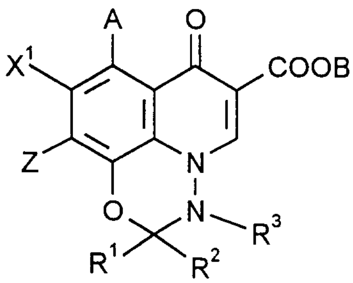

(I),

in welcher

A für Amino oder Alkylamino steht,

und die übrigen Reste die in der Beschreibung angegebene Bedeutung haben, Verfahren zu ihrer Herstellung und ihre Verwendung in antibakteriellen Mitteln.

EP 0 683 169 A1

Die Erfindung betrifft neue 8-Amino-pyrido[1,2,3-d,e][1,3,4]benzoxadiazinderivate, Verfahren zu ihrer Herstellung sowie diese enthaltende antibakterielle Mittel.

Es ist bereits bekannt geworden, daß Pyridobenzoxadiazincarbonsäuren antibakteriell wirksam sind. Beispiele hierfür finden sich in EP-O 259 804, EP-O 343 524 und im European Journal of Medicinal Chemistry 26, 889 (1991).

Die vorliegende Erfindung betrifft:

1. Neue 8-Amino-pyrido[1,2,3-d,e][1,3,4]benzoxadiazinderivate der allgemeinen Formel (I)

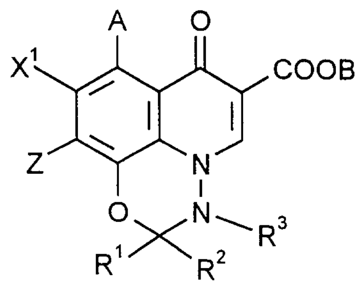

(I),

in welcher

- A für Amino oder Alkylamino steht,
- $R^1$ für Wasserstoff oder gegebenenfalls durch Hydroxy oder Halogen substituiertes $C_1$-$C_3$-Alkyl steht,
- $R^2$ unabhängig von $R^1$ für Wasserstoff oder Methyl steht,
- $R^3$ für Wasserstoff oder $C_1$-$C_4$-Alkyl steht,
- B für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- $X^1$ für Wasserstoff oder Halogen steht,
- Z für Reste der Strukturen

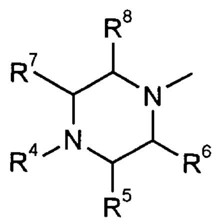
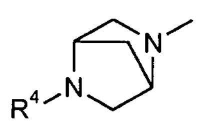
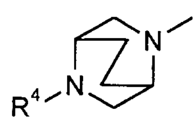
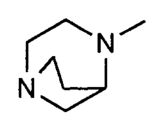

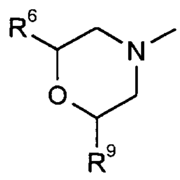
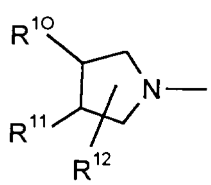
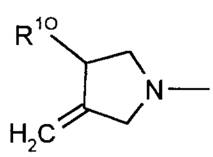
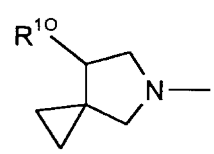

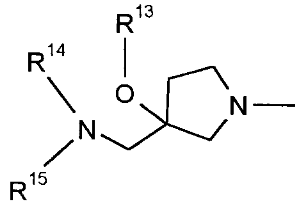
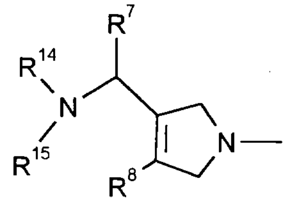
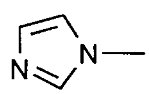
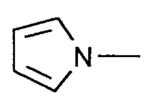

steht, worin

- $R^4$ für Wasserstoff, gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,
- $R^5$ für Wasserstoff, Methyl, Phenyl, Thienyl oder Pyridyl steht,
- $R^6$ für Wasserstoff oder $C_{1-4}$-Alkyl steht,
- $R^7$ für Wasserstoff oder $C_{1-4}$-Alkyl steht,
- $R^8$ für Wasserstoff oder $C_{1-4}$-Alkyl steht,
- $R^9$ für Wasserstoff Methyl oder -$CH_2$-$NR^7R^8$ steht,
- $R^{10}$ für Wasserstoff, $C_{1-4}$-Alkyl, Amino, gegebenenfalls durch Hydroxy substituiertes Alkyl- oder Dialkylamino mit 1 oder 2 C-Atomen im Alkylteil, Amino-$C_{1-4}$-Alkyl, $C_{1-4}$-Alkylamino-$C_{1-4}$-Alkyl oder 1-Imidazolyl steht,
- $R^{11}$ für Wasserstoff, Hydroxy, Methoxy, Methylthio, Halogen, Methyl, Hydroxymethyl steht,
- $R^{12}$ für Wasserstoff oder Methyl steht,
- $R^{13}$ für Wasserstoff oder $C_{1-4}$-Alkyl steht,
- $R^{14}$ für Wasserstoff oder $C_{1-4}$-Alkyl steht und
- $R^{15}$ für Wasserstoff oder $C_{1-4}$-Alkyl steht,

in Form von Racematen oder als enantiomerenreine Verbindungen, ihre pharmazeutisch verwendbaren Hydrate und Säureadditionssalze sowie ihre Salze mit Basen.

2. Verfahren zur Herstellung der Verbindungen der Formel (I)

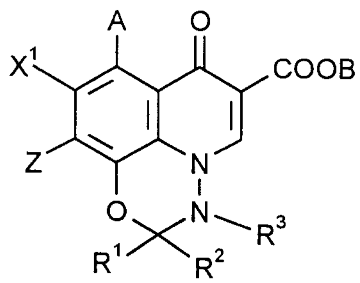

(I),

in welcher

- A für Amino oder Alkylamino steht,
- $R^1$ für Wasserstoff oder gegebenenfalls durch Hydroxy oder Halogen substituiertes $C_1$-$C_3$-Alkyl steht,
- $R^2$ unabhängig von $R^1$ für Wasserstoff oder Methyl steht,
- $R^3$ für Wasserstoff oder $C_1$-$C_4$-Alkyl steht,
- B für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- $X^1$ für Wasserstoff oder Halogen steht,
- Z für Reste der Strukturen

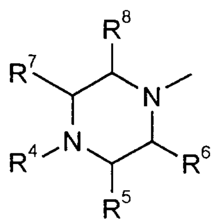 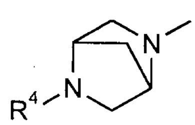 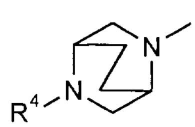 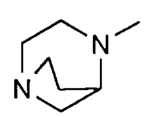

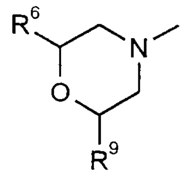 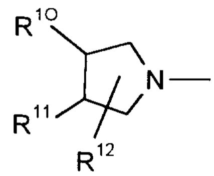 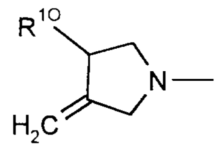 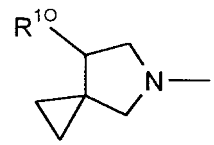

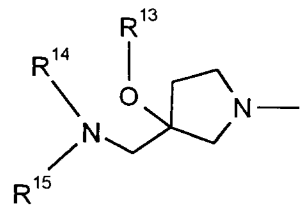 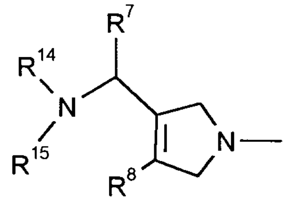 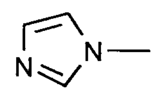 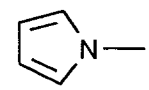

steht, worin

$R^4$ für Wasserstoff, gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,

$R^5$ für Wasserstoff, Methyl, Phenyl, Thienyl oder Pyridyl steht,

$R^6$ für Wasserstoff oder $C_{1-4}$-Alkyl steht,

$R^7$ für Wasserstoff oder $C_{1-4}$-Alkyl steht,

$R^8$ für Wasserstoff oder $C_{1-4}$-Alkyl steht,

$R^9$ für Wasserstoff, Methyl oder -$CH_2$-$NR^7R^8$ steht,

$R^{10}$ für Wasserstoff, $C_{1-4}$-Alkyl, Amino, gegebenenfalls durch Hydroxy substituiertes Alkyl- oder Dialkylamino mit 1 oder 2 C-Atomen im Alkylteil, Amino-$C_{1-4}$-alkyl, $C_{1-4}$-Alkylamino-$C_{1-4}$-Alkyl oder 1-Imidazolyl steht,

$R^{11}$ für Wasserstoff, Hydroxy, Methoxy, Methylthio, Halogen, Methyl, Hydroxymethyl steht,

$R^{12}$ für Wasserstoff oder Methyl steht,

$R^{13}$ für Wasserstoff oder $C_{1-4}$-Alkyl steht,

$R^{14}$ für Wasserstoff oder $C_{1-4}$-Alkyl steht und

$R^{15}$ für Wasserstoff oder $C_{1-4}$-Alkyl steht,

dadurch gekennzeichnet, daß man Verbindungen der Formel (II)

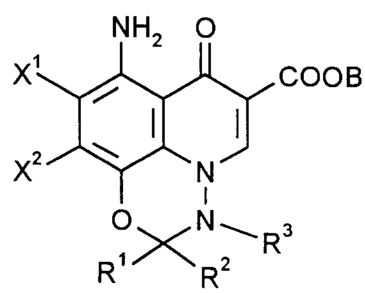

(II),

in welcher

$R^1$, $R^2$, $R^3$, B und $X^1$ die oben angegebene Bedeutung haben und

$X^2$ für Halogen, insbesondere Fluor oder Chlor, steht,

mit Verbindungen der Formel ((III)

Z-H (III),

in welcher

Z die oben angegebene Bedeutung hat,

gegebenenfalls in Gegenwart von Säureakzeptoren umsetzt.

3. Verbindungen der Formel (II)

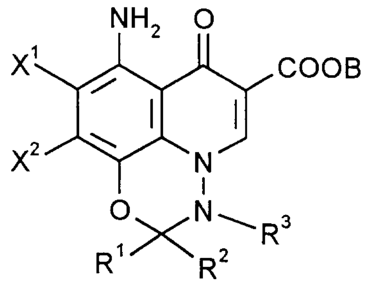

(II),

in welcher

$R^1$, $R^2$, $R^3$, B und $X^1$ die oben angegebene Bedeutung haben und

$X^2$ für Halogen, insbesondere Fluor oder Chlor, steht.

4. Verfahren zur Herstellung der Verbindungen der Formel (II)

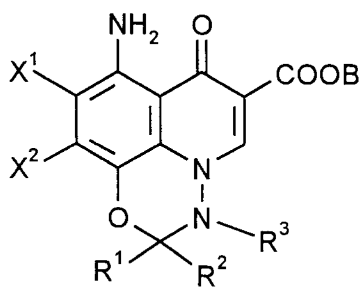

(II),

in welcher

$R^1$, $R^2$, $R^3$, B und $X^1$ die oben angegebene Bedeutung haben und

$X^2$ für Halogen, insbesondere Fluor oder Chlor, steht,

dadurch gekennzeichnet, daß man Verbindungen der Formel (IV)

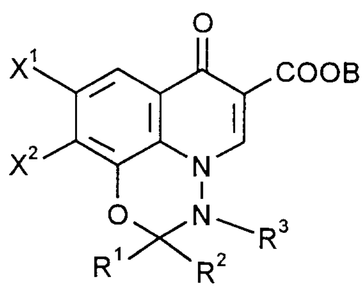

(IV),

in welcher

$R^1$, $R^2$, $R^3$, B, $X^1$ und $X^2$ die oben angegebene Bedeutung haben,

mit Nitrierungsreagenzien wie Salpetersäure und Nitraten in einem Lösungsmittel wie zum Beispiel Wasser, Schwefelsäure, Essigsäure, Acetanhydrid oder deren Gemische bei -50 °C bis 200 °C, vorzugsweise bei -20 °C bis 100 °C, umsetzt und anschließend die erhaltenen Nitroverbindungen reduziert.

Die erfindungsgemäßen Verbindungen eignen sich als Wirkstoffe für die Human- und Veterinärmedizin.

Bevorzugt sind Verbindungen der Formel (I),

in welcher

A für Amino steht,

$R^1$ für Wasserstoff oder gegebenenfalls durch Hydroxy substituiertes $C_1$-$C_3$-Alkyl steht,

$R^2$ unabhängig von $R^1$ für Wasserstoff oder Methyl steht,

$R^3$ für Wasserstoff Methyl oder Ethyl steht,

B für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-

methyl steht,

$X^1$ für Wasserstoff, Fluor oder Chlor steht,

Z für Reste der Strukturen

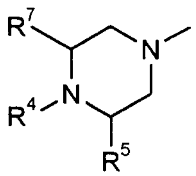
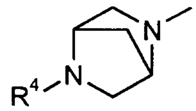
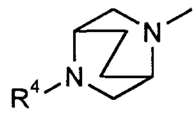
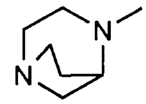
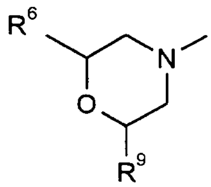
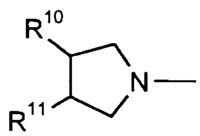
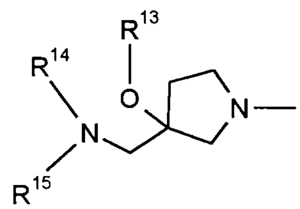
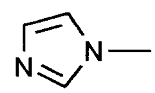

steht, worin

$R^4$ für Wasserstoff, gegebenenfalls durch Hydroxy substituiertes geradkettiges oder verzweigtes $C_1$-$C_3$-Alkyl, Oxalkyl mit 1 bis 4 C-Atomen steht,

$R^5$ für Wasserstoff Methyl oder Phenyl steht,

$R^6$ für Wasserstoff oder Methyl steht,

$R^7$ für Wasserstoff oder Methyl steht,

$R^9$ für Wasserstoff, Methyl oder $-CH_2NH_2$ steht,

$R^{10}$ für Wasserstoff, Methyl, Amino, Methylamino, Dimethylamino, Aminomethyl, Methylaminomethyl oder Ethylaminomethyl steht,

$R^{11}$ für Wasserstoff Hydroxy, Methyl, Fluor, Methyl oder Hydroxyn thyl steht,

$R^{13}$ für Wasserstoff oder Methyl steht,

$R^{14}$ für Wasserstoff oder Methyl steht,

$R^{15}$ für Wasserstoff oder Methyl steht,

und deren pharmazeutisch verwendbaren Hydrate und Säureadditionssalze sowie ihre Alkali-, Erdalkali-, Silber- und Guanidiniumsalze.

Besonders bevorzugt sind Verbindungen der Formel (I),

in welcher

A für Amino steht,

$R^1$ für Wasserstoff oder Methyl, das gegebenenfalls durch Hydroxy substituiert ist, steht,

$R^2$ für Wasserstoff steht,

$R^3$ für Methyl oder Ethyl steht,

B für Wasserstoff Methyl oder Ethyl steht,

$X^1$ für Fluor steht,

Z für Reste der Strukturen

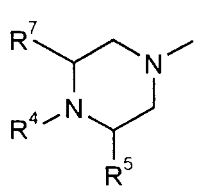
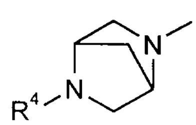
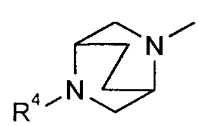

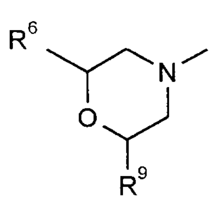
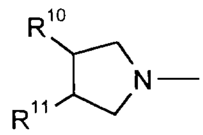
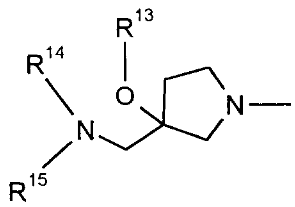
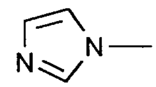

steht, worin

$R^4$ für Wasserstoff, Methyl, gegebenenfalls durch Hydroxy substituiertes Ethyl steht,
$R^5$ für Wasserstoff oder Methyl steht,
$R^6$ für Wasserstoff oder Methyl steht,
$R^7$ für Wasserstoff oder Methyl steht,
$R^9$ für Wasserstoff, Methyl oder $-CH_2NH_2$ steht,
$R^{10}$ für Wasserstoff, Methyl, Amino, Methylamino, Aminomethyl oder Ethylaminomethyl steht,
$R^{11}$ für Wasserstoff Hydroxy oder Methoxy steht,
$R^{13}$ für Wasserstoff oder Methyl steht,
$R^{14}$ für Wasserstoff oder Methyl steht,
$R^{15}$ für Wasserstoff oder Methyl steht,

und deren pharmazeutisch verwendbaren Hydrate und Säureadditionssalze sowie die Alkali-, Erdalkali-, Silber- und Guanidiniumsalze der zugrundeliegenden Carbonsäuren.

Verwendet man beim Verfahren zur Herstellung der Verbindungen der Formel (I) beispielsweise 8-Amino-9,10-difluor-3-methyl-7-oxo-2,3-dihydro-7H-pyrido[1,2,3]-[1,3,4]benzoxadiazin-6-carbonsäure und Piperazin, so kann der Reaktionsverlauf durch das folgende Formelschema wiedergegeben werden:

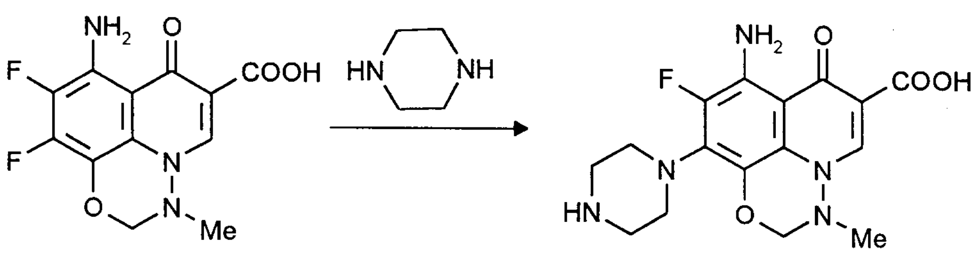

Die als Ausgangsverbindungen verwendeten Verbindungen der Formel (II) sind neu. Sie können hergestellt werden, indem man Verbindungen der Formel (IV)

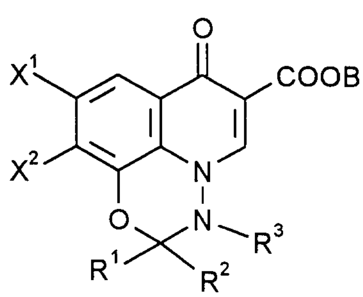
(IV),

in welcher

$R^1$, $R^2$, $R^3$, B, $X^1$ und $X^2$ die oben angegebene Bedeutung haben,

mit Nitrierungsreagenzien wie Salpetersäure und Nitraten in einem Lösungsmittel wie zum Beispiel Wasser, Schwefelsäure, Essigsäure, Acetanhydrid oder deren Gemischen bei -50 °C bis 200 °C, vorzugsweise bei -20 °C bis 100 °C umsetzt und anschließend die erhaltenen Nitroverbindungen reduziert.

Zur Reduktion der Nitrogruppe können Metallhydride, Übergangsmetalle und Übergangsmetallsalze eingesetzt werden; bevorzugt wird Wasserstoff in Gegenwart von Katalysatoren wie zum Beispiel Palladium-Kohle, Raney-Nickel und Platin verwendet. Als Lösungsmittel können zum Beispiel Wasser, Salzsäure,

Alkohole, Essigsäure oder auch deren Gemische verwendet werden.

Verbindungen der Formel (II) können auch nach folgendem Reaktionsschema, in dem $R^1$, $R^2$, $R^3$, B, $X^1$ und $X^2$ die oben angegebene Bedeutung besitzen, hergestellt werden:

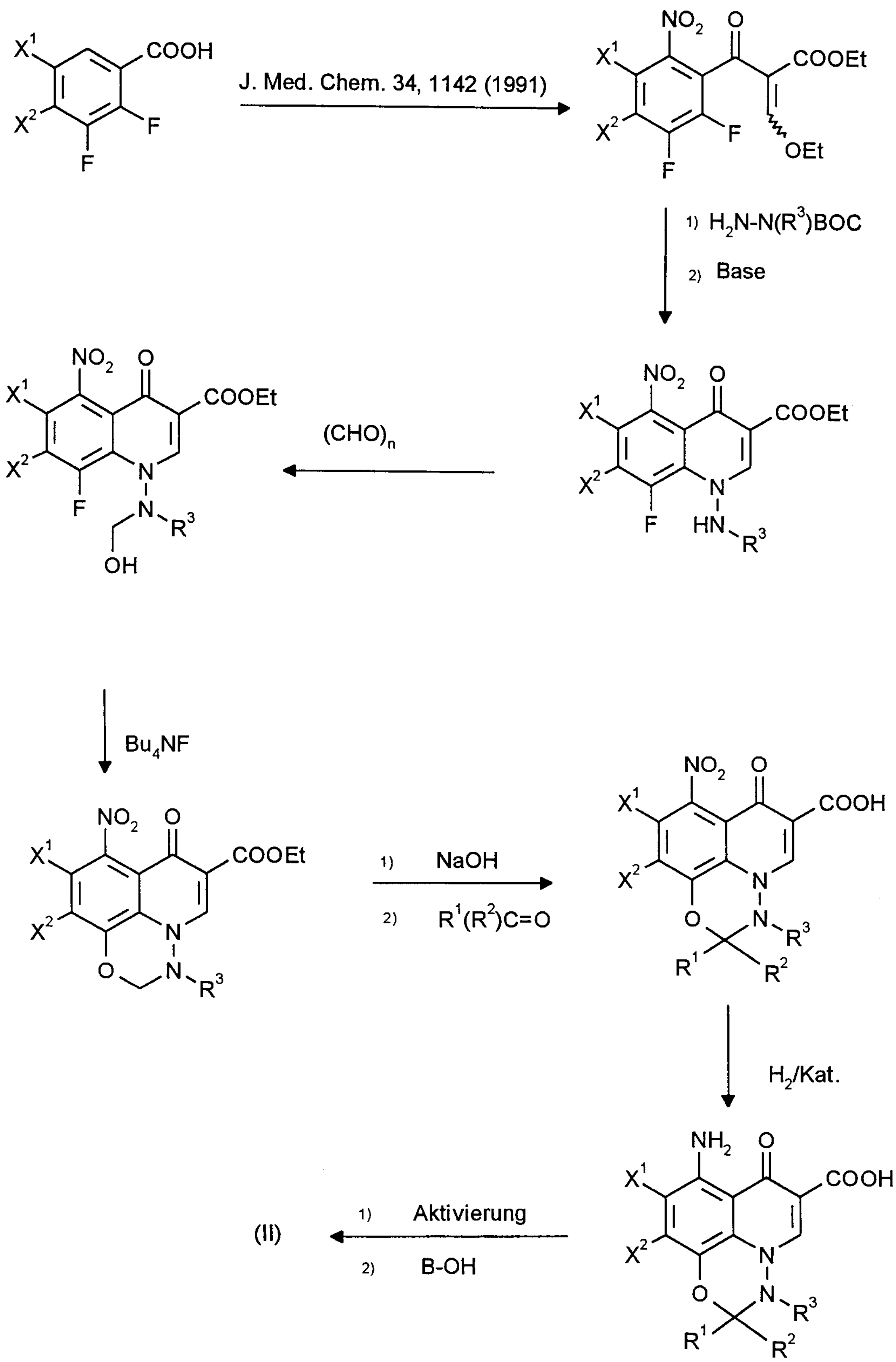


Sie können gegebenenfalls als Racemate, Enantiomere oder reine Diastereomere eingesetzt werden.

Als Beispiele seien genannt:
8-Amino-9,10-difluor-3-methyl-7-oxo-2,3-dihydro-7H-pyrido[1,2,3-d,e][1,3,4]benzoxadiazin-6-carbonsäure,
8-Amino-9,10-difluor-2,3-dimethyl-7-oxo-2,3-dihydro-7H-pyrido[1,2,3-d,e][1,3,4]-benzoxadiazin-6-carbonsäure,
8-Amino-9,10-difluor-2-(hydroxymethyl)-3-methyl-7-oxo-2,3-dihydro-7H-pyrido-[1,2,3-d,e]-[1,3,4]-benzoxadiazin-6-carbonsäure,
8-Amino-9,10-difluor-3-ethyl-7-oxo-2,3-dihydro-7H-pyrido[1,2,3-d,e][1,3,4]benzoxadiazin-6-carbonsäure,
8-Amino-9,10-difluor-3-methyl-7-oxo-2,3-dihydro-7H-pyrido[1,2,3-d,e][1,3,4]benzoxadiazin-6-carbonsäureethylester.

Die als Ausgangsverbindungen verwendeten Amine der Formel (III) sind bekannt. Chirale Amine können sowohl als Racemate, als auch als enantiomeren- oder diastereomerenreine Verbindungen eingesetzt werden.

Als Beispiele seien genannt:
Piperazin,
1-Methylpiperazin,
2-Ethylpiperazin,
2-Methylpiperazin,
2,6-Dimethylpiperazin,
1-Cyclopropylpiperazin,
2-Phenylpiperazin,
1,2-Dimethylpiperazin,
2,5-Diazabicyclo[2.2.1]heptan,
2-Methyl-2,5-diazabicyclo[2.2.1]heptan,
2,5-Diazabicyclo[2.2.2]octan,
2-Methyl-2,5-diazabicyclo[2.2.2]octan,
1,4-Diazabicyclo[3.2.1]octan,
Morpholin,
2,6-Dimethylmorpholin,
2-Aminomethylmorpholin,
Imidazol,
Pyrrol,
3-Aminomethyl-2,5-dihydropyrrol,
3-Aminomethyl-4-methyl-2,5-dihydropyrrol,
Pyrrolidin,
3-Methylpyrrolidin,
3-Amino-4-methylenpyrrolidin,
7-Amino-5-azaspiro[2.4]heptan,
3-Amino-pyrrolidin,
3-tert.-Butoxycarbonylamino-pyrrolidin,
3-Methylamino-pyrrolidin,
3-Dimethylamino-pyrrolidin,
3-Aminomethyl-pyrrolidin,
3-tert-Butoxycarbonylaminomethyl-pyrrolidin,
4-Chlor-3-tert.-butoxycarbonylaminomethyl-pyrrolidin,
3-tert.-Butoxycarbonylaminomethyl-3-methyl-pyrrolidin,
3-tert-Butoxycarbonylamino-4-methyl-pyrrolidin,
3-tert-Butoxycarbonylaminomethyl-3-methoxy-pyrrolidin,
3-Methylaminomethyl-pyrrolidin,
3-Ethylaminomethyl-pyrrolidin,
4-tert.-Butoxycarbonylamino-2-methyl-pyrrolidin,
2-Methyl-3-methylamino-pyrrolidin,
2-Methyl-4-methylamino-pyrrolidin,
3-(2-Hydroxyethylamino)-pyrrolidin,
3-Hydroxy-pyrrolidin,
3-Hydroxymethyl-pyrrolidin,
4-Amino-3-hydroxy-pyrrolidin,
3-Hydroxy-4-methylamino-pyrrolidin,
3-tert.-Butoxycarbonylamino-4-methoxy-pyrrolidin,

3-Methylaminomethyl-3-hydroxy-pyrrolidin,
3-Dimethylaminomethyl-3-hydroxy-pyrrolidin,
3-Diethylaminomethyl-3-hydroxy-pyrrolidin,
3-tert.-Butylaminomethyl-3-hydroxy-pyrrolidin,
3-Methylamino-4-hydroxymethyl-pyrrolidin,
4-Methoxy-3-methylamino-pyrrolidin,
3-Methoxy-3-methylaminomethyl-pyrrolidin,
3-Amino-4-methoxy-2-methyl-pyrrolidin,
3-Methyl-4-tert.-butoxycarbonylaminomethyl-pyrrolidin und
3-Methoxy-4-tert.butoxycarbonylaminomethyl-pyrrolidin,
Im einzelnen seien folgende Verbindungen der Formel (Ia) genannt:

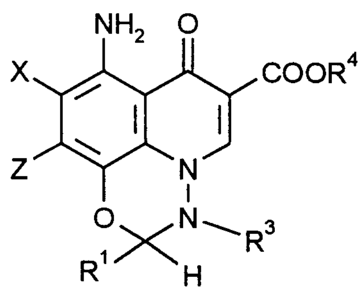

(Ia)

| $R^1$ | $R^3$ | $R^4$ | Z | X |
|---|---|---|---|---|
| H | Me | H | 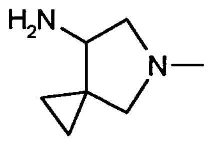 | F |
| H | Me | H | 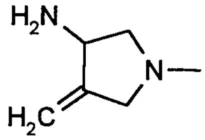 | F |
| H | Me | H | 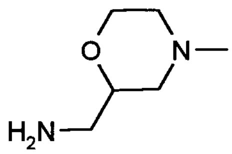 | F |
| H | Me | Et | 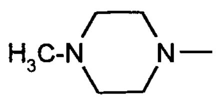 | F |
| H | Et | Et | 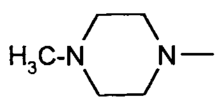 | F |

Me = Methyl
Et = Ethyl

Fortsetzung:

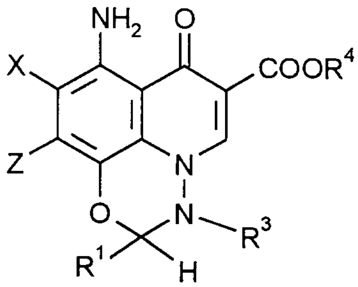

(Ia)

| $R^1$ | $R^3$ | $R^4$ | Z | X |
|---|---|---|---|---|
| Me | Me | H | 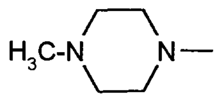 | F |
| Me | Me | H | 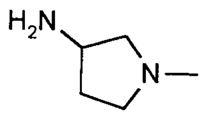 | F |
| $-CH_2OH$ | Me | H | 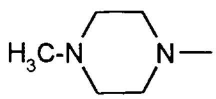 | F |
| H | H | H | 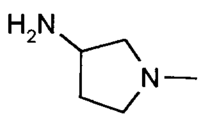 | F |
| $CH_3$ | $CH_3$ | H | 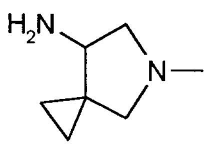 | F |

Fortsetzung:

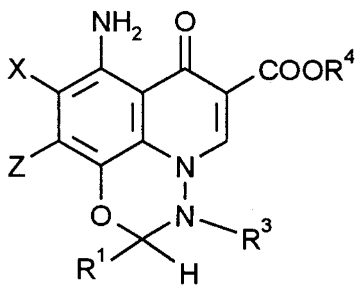

(Ia)

| $R^1$ | $R^3$ | $R^4$ | Z | X |
|---|---|---|---|---|
| H | $CH_3$ | Ethyl | 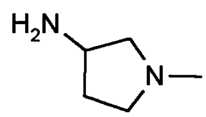 | F |
| H | $CH_3$ | $-CH_2-CH_2-NH_2$ | 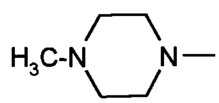 | F |
| H | $CH_3$ | $-CH_2-CH_2-OCH_3$ | 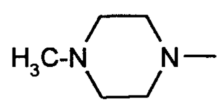 | F |
| $CH_3$ | $CH_3$ | H | 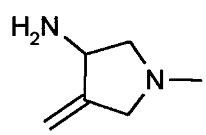 | F |

Fortsetzung:

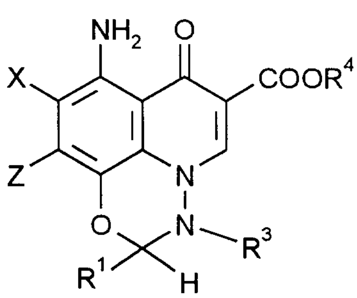

(Ia)

| $R^1$ | $R^3$ | $R^4$ | Z | X |
|---|---|---|---|---|
| H | Me | H | 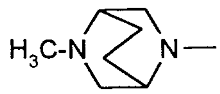 | F |
| H | Me | H | 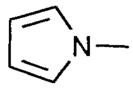 | F |
| H | Me | H | 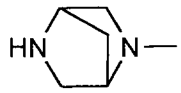 | F |
| H | Et | H | 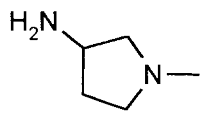 | F |
| $CH_2OH$ | Me | H | 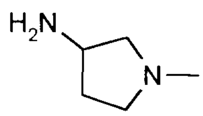 | F |

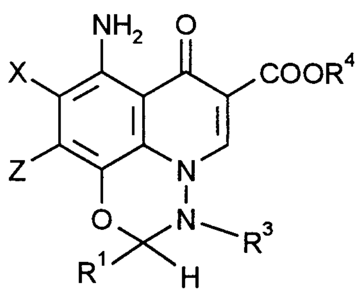

(Ia)

| $R^1$ | $R^3$ | $R^4$ | Z | X |
|---|---|---|---|---|
| H | Me | H | 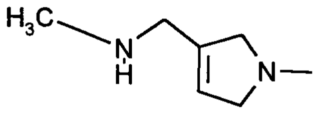 | F |
| H | Me | H | 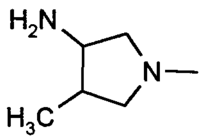 | F |
| H | Me | H | 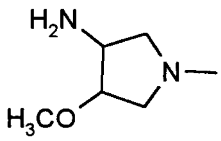 | F |
| H | Me | H | 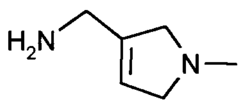 | F |
| H | Me | H | 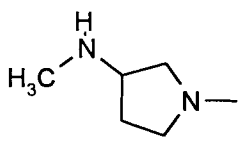 | F |

Fortsetzung:

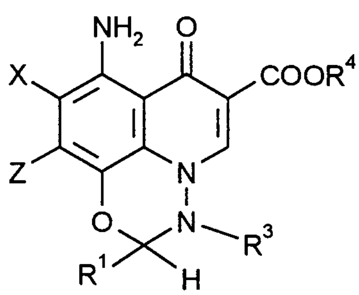

(Ia)

| $R^1$ | $R^3$ | $R^4$ | Z | X |
|---|---|---|---|---|
| H | Me | H | 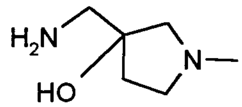 | F |
| H | Me | H | 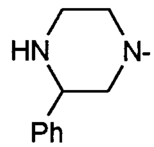 | F |
| H | Me | H | 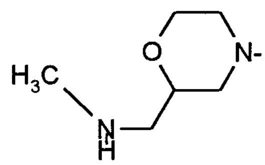 | F |
| H | Me | H | 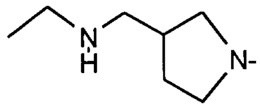 | F |
| $-CH_2OH$ | Me | H | 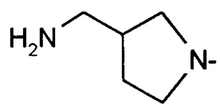 | F |

Ph = Phenyl

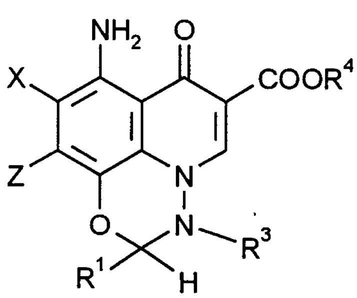

(Ia)

| $R^1$ | $R^3$ | $R^4$ | Z | X |
|---|---|---|---|---|
| $-CH_2OH$ | Me | H | 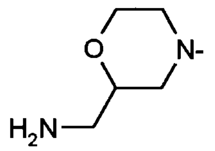 | F |
| $-CH_2OH$ | Me | H | 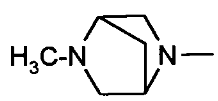 | F |
| H | Et | H | 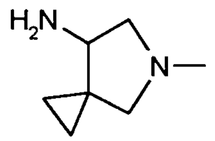 | F |

Die Umsetzung von Verbindungen der Formel (II) mit Verbindungen der Formel (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können wahrend der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester werden durch Umsetzung eines Alkalisalzes der Zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100 °C, vorzugsweise 0 bis 50 °C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäue, Embonsäure, Glutaminsäure oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen unterhalb von Konzentrationen ähnlicher Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Micrococcus luteus und Acinetobacter beobachtet werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Injektions- und orale verabreichbare Lösungen, Suspensionen und Emulsionen, ferner Pasten, Salben, Gele, Cremes, Lotions, Puder und Sprays genannt.

Die Wirkstoffe eignen sich bei günstiger Warmblütertoxizität bevorzugt zur Bekämpfung von bakteriellen Erkrannkungen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien sowie gegen resistente und normal sensible Stämme wirksam. Durch die Bekämpfung der bakteriellen Erkrankungen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-$\beta$-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Die minimalen Hemmkonzentrationen (MHK) der erfindungsgemäßen Verbindungen wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37 °C bebrütet, und das Keimwachstum wurde nach ca. 20 Stunden abgelesen. Der MHK-Wert ($\mu$g/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen aufgeführt.

Tabelle

| MHK-Werte | | | | | |
|---|---|---|---|---|---|
| Spezies | Stamm | Beispiel Nr. | | | |
| | | 2 | 3 | 5 | 10 |
| E. coli | Neumann<br>ATCC 25922 | 0.015<br>0.015 | 0.06<br>0.06 | 0.015<br>0.03 | 0.06<br>0.06 |
| Klebsiella pneumoniae | 8085 | 0.06 | 0.12 | 0.25 | 0.12 |
| Acinetobacter | 14068 | 0.015 | 0.015 | 0.015 | 0.015 |
| Micrococcus luteus | 9341 | 0.015 | 0.015 | 0.015 | 0.015 |

## Herstellung der Wirkstoffe

### Beispiel 1

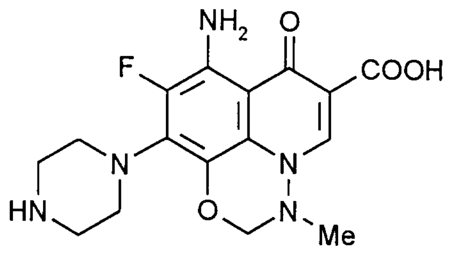

**8-Amino-9-fluor-3-methyl-10-(1-piperazinyl)-7-oxo-2,3-dihydro-7H-pyrido-[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 58 mg (0,673 mmol) Piperazin in 3 ml DMSO zwei Stunden unter Argon auf 120 ° C erwärmt. Die Mischung wird im Hochvakuum eingeengt, der Rückstand aus Ethanol umkristallisiert und getrocknet.
Ausbeute: 112 mg (92 % der Theorie)
Schmelzpunkt: >300 ° C

### Beispiel 2

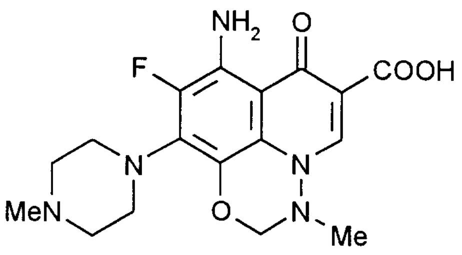

**8-Amino-9-fluor-3-methyl-10-(4-methyl-1-piperazinyl)-7-oxo-2,3-dihydro-7H-pyrido[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 67 mg (0,673 mmol) 1-Methylpiperazin in 3 ml DMSO vier Stunden unter Argon auf 120 ° C erwärmt. Die Mischung wird im Hochvakuum eingeengt, der Rückstand aus Ethanol umkristallisiert und getrocknet.
Ausbeute: 100 mg (79 % der Theorie)
Schmelzpunkt: >300 ° C

**Beispiel 3**

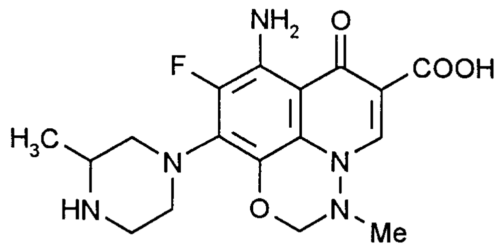

**8-Amino-9-fluor-3-methyl-10-(3-methyl-1-piperazinyl)-7-oxo-2,3-dihydro-7H-pyrido[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazon-6-carbonsäure werden mit 67 mg (0,673 mmol) 2-Methylpiperazin in 3 ml DMSO vier Stunden unter Argon auf 120 °C erwärmt. Die Mischung wird im Hochvakuum eingeengt, der Rückstand aus Ethanol umkristallisiert und getrocknet.
Ausbeute: 96 mg (76 % der Theorie)
Schmelzpunkt: 280 °C (unter Zersetzung)

**Beispiel 4**

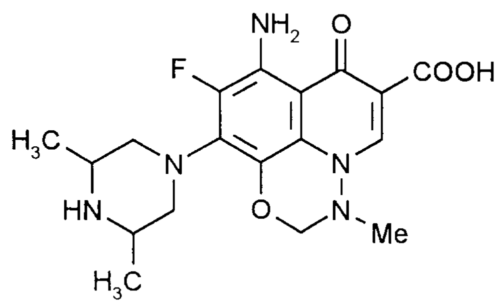

**8-Amino-9-fluor-3-methyl-10-(3,5-dimethyl-1-piperazinyl)-7-oxo-2,3-dihydro-7H-pyrido[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 77 mg (0,673 mmol) 2,6-Dimethylpiperazin in 3 ml DMSO zwei Stunden unter Argon auf 120 °C erwärmt. Die Mischung wird im Hochvakuum eingeengt, der Rückstand aus Ethanol umkristallisiert und getrocknet.
Ausbeute: 108 mg (82 % der Theorie)
Schmelzpunkt: 280 °C

**Beispiel 5**

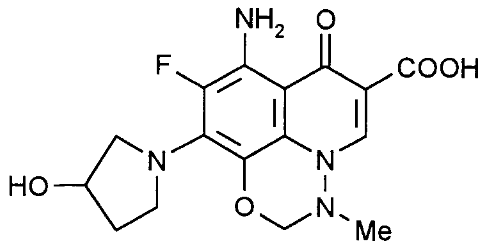

**8-Amino-9-fluor-3-methyl-10-(3-hydroxy-1-pyrrolidinyl)-7-oxo-2,3-dihydro-7H-pyrido[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 59 mg (0,673 mmol) 3-Hydroxypyrrolidin in 3 ml DMSO zwei Stunden unter Argon auf 120 °C erwärmt. Die Mischung wird im Hochvakuum eingeengt, der Rückstand aus Ethanol umkristallisiert und getrocknet.
Ausbeute: 93 mg (76 % der Theorie)
Schmelzpunkt: >300 °C

**Beispiel 6**

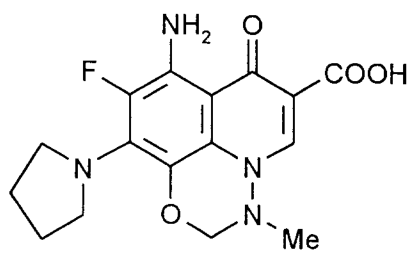

**8-Amino-9-fluor-3-methyl-10-(1-pyrrolidinyl)-7-oxo-2,3-dihydro-7H-pyrido-[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 48 mg (0,673 mmol) Pyrrolidin in 3 ml DMSO zwei Stunden unter Argon auf 120 °C erwärmt. Die Mischung wird im Hochvakuum eingeengt, der Rückstand aus Ethanol umkristallisiert und getrocknet.
Ausbeute: 80 mg (68 % der Theorie)
Schmelzpunkt: >300 °C

**Beispiel 7**

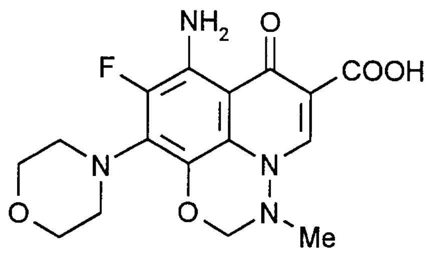

**8-Amino-9-fluor-3-methyl-10-(4-morpholinyl)-7-oxo-2,3-dihydro-7H-pyrido-[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 59 mg (0,673 mmol) Morpholin in 3 ml DMSO vier Stunden unter Argon auf 120 ° C erwärmt. Die Mischung wird im Hochvakuum eingeengt, der Rückstand aus Ethanol umkristallisiert und getrocknet.
Ausbeute: 99 mg (81 % der Theorie)
Schmelzpunkt: >300 ° C

**Beispiel 8**

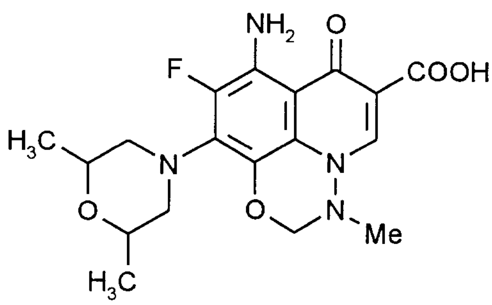

**8-Amino-9-fluor-3-methyl-10-(2,6-dimethyl-4-morpholinyl)-7-oxo-2,3-dihydro-7H-pyrido[1,2,3-d,e]-[1,3,4]benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 78 mg (0,673 mmol) 2,6-Dimethylmorpholin in 3 ml DMSO vier Stunden unter Argon auf 120 ° C erwärmt. Die Mischung wird im Hochvakuum eingeengt, der Rückstand aus Ethanol umkristallisiert und getrocknet.
Ausbeute: 88 mg (81 % der Theorie)
Schmelzpunkt: >300 ° C

**Beispiel 9**

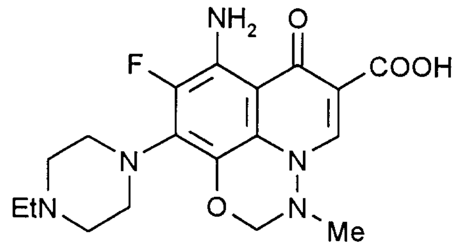

**8-Amino-9-fluor-3-methyl-10-(4-ethyl-piperazinyl)-7-oxo-2,3-dihydro-7H-pyrido[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 77 mg (0,673 mmol) 1-Ethylpiperazin in 3 ml DMSO zwei Stunden unter Argon auf 120 °C erwärmt. Die Mischung wird im Hochvakuum eingeengt, der Rückstand aus Ethanol umkristallisiert und getrocknet.
Ausbeute: 118 mg (90 % der Theorie)
Schmelzpunkt: >300 °C

**Beispiel 10**

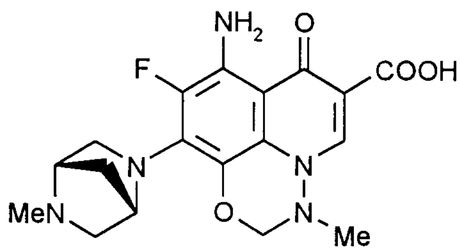

**8-Amino-9-fluor-3-methyl-10-(1S,4S)-5-methyl-2,5-diazabicyclo[2.2.1]hept-2-yl)-7-oxo-2,3-dihydro-7H-pyrido[1,2,3-d,e][1,3,4]benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 76 mg (0,673 mmol) (1S,4S)-5-Methyl-2,5-diazabicyclo[2.2.1]heptan in 3 ml DMSO zwei Stunden unter Argon auf 120 °C erwärmt. Die Mischung wird im Hochvakuum eingeengt, der Rückstand aus Ethanol umkristallisiert und getrocknet.
Ausbeute: 112 mg (85 % der Theorie)
Schmelzpunkt: 270 °C (unter Zersetzung)

**Beispiel 11**

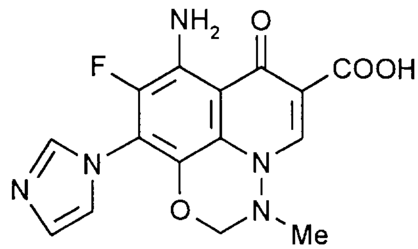

**8-Amino-9-fluor-3-methyl-10-(1-imidazolyl)-7-oxo-2,3-dihydro-7H-pyrido[1,2,3-d,e][1,3,4]-benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 46 mg (0,673 mmol) Imidazol in 3 ml DMSO sechs Stunden unter Argon auf 120 °C erwärmt. Die Mischung wird im Hochvakuum eingeengt, der Rückstand aus Ethanol umkristallisiert und getrocknet.
Ausbeute: 60 mg (76 % der Theorie)
Schmelzpunkt: >300 °C

**Beispiel 12**

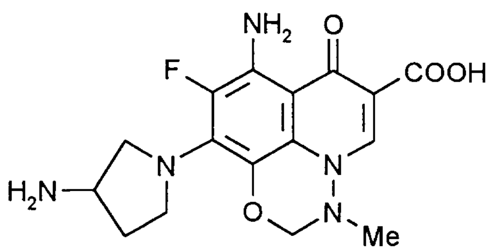

**8-Amino-9-fluor-3-methyl-10-(3-amino-1-pyrrolidinyl)-7-oxo-2,3-dihydro-7H-pyrido[1,2,3-d,e][1,3,4]-benzoxadiazin-6-carbonsäure-Trifluoracetat**

a) 8-Amino-9-fluor-methyl-10-(3-tert.-butoxycarbonylamino)-1-pyrrolidinyl)-7-oxo-2,3-dihydro-7H-pyrido[1,2,3-d,e][1,3,4]benzoxadiazin-6-carbonsäure

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 125 mg (0,673 mmol) 3-(tert.-Butoxycarbonylamino)-pyrrolidin in 3 ml DMSO zwei Stunden unter Argon auf 120 °C erwärmt. Die Mischung wird im Hochvakuum eingeengt, der Rückstand aus Ethanol umkristallisiert und getrocknet. Ausbeute: 122 mg (78 % der Theorie) Schmelzpunkt: 275 °C

b) 8-Amino-9-fluor-methyl-10-(3-amino-1-pyrrolidinyl)-7-oxo-2,3-dihydro-7H-pyrido[1,2,3-d,e][1,3,4]-benzoxadiazin-6-carbonsäure-Trifluoracetat

80 mg (0,17 mmol) des Produktes aus Stufe a) werden in 2 ml Trifluoressigsäure unter Eiskühlung gelöst, die Lösung im Vakuum eingeengt, der Rückstand aus Ethanol kristallisiert und getrocknet.
Ausbeute: 70 mg (86 % der Theorie)
Schmelzpunkt: 260 °C

**Beispiel 13**

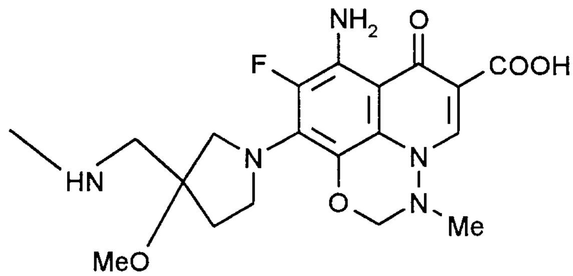

**8-Amino-9-fluor-3-methyl-10-(3-methoxy-3-methylaminomethyl-1-pyrrolidin-yl)-7-oxo-2,3-dihydro-7H-pyrido[1,2,3-d,e][1,3,4]benzoxadiazin-6-carbonsäure**

100 mg (0,337 mmol) 8-Amino-9,10-difluor-3-methyl-7-oxo-2,3-dihydro-7H-pyrido[1,2,3-d,e][1,3,4]-benzoxadiazin-6-carbonsäure werden mit 97 mg (0,673 mmol) 3-Methoxy-3-methylaminomethyl-pyrrolidin in 3 ml DMSO zwei Stunden unter Argon auf 120 °C erwärmt. Die Mischung wird im Hochvakuum eingeengt, der Rückstand aus Ethanol umkristallisiert und getrocknet.
Ausbeute: 106 mg (75 % der Theorie)
Schmelzpunkt: 238-240 °C

**Herstellung von Ausgangsverbindungen**

**9,10-Difluor-3-methyl-8-nitro-7-oxo-2,3-dihydro-7H-pyrido[1,2,3-d,e][1,3,4]-benzoxadiazin-6-carbonsäure**

Zu 7,0 g (24,8 mmol) 9,10-Difluor-3-methyl-7-oxo-2,3-dihydro-7H-pyrido[1,2,3-d,e][1,3,4]-benzoxadiazin-6-carbonsäure, gelöst in 90 ml konzentrierter Schwefelsäure, gibt man portionsweise 3,7 g (36,6 mmol) Kaliumnitrat und läßt eine Stunde bei Raumtemperatur rühren. Die Reaktionsmischung wird auf 270 ml Eiswasser gegeben. Der resultierende Niederschlag wird abgesaugt, mit Wasser gewaschen und getrocknet.
Ausbeute: 6,9 g (85 % der Theorie)
Schmelzpunkt: >300 °C

**8-Amino-9,10-difluor-3-methyl-7-oxo-2,3-dihydro-7H-pyrido[1,2,3-d,e][1,3,4]-benzoxadiazin-6-carbonsäure**

4,0 g (12,2 mmol) 9,10-Difluor-3-methyl-8-nitro-7-oxo-2,3-dihydro-7H-pyrido-[1,2,3-d,e][1,3,4]-benzoxadiazin-6-carbonsäure und 1,2 g Palladium auf Aktivkohle (10 % Palladium) werden in 280 ml Ethanol suspendiert und zwei Tage unter Normaldruck bei Raumtemperatur hydriert. Die Reaktionsmischung wird mit 280 ml Wasser versetzt und anschließend mit 2N Natronlauge auf pH 10-11 eingestellt. Der Hydrierkatalysator wird abfiltriert und das Filtrat mit 2N Salzsäure auf pH 5-6 eingestellt. Der resultierende Niederschlag wird abfiltriert, mit Methanol gewaschen und getrocknet (Fraktion A). Der abfiltrierte Hydrierkatalysator wird dreimal in je 100 l Dimethylformamid (DMF) für eine Stunde unter Rückfluß erhitzt und anschließend wieder abfiltriert. Die vereinigten DMF-Lösungen werden im Vakuum eingeengt und getrocknet (Fraktion B).
Ausbeute: 3,0 g (83 % der Theorie)
Schmelzpunkt: >300 °C

## Patentansprüche

**1.** 8-Amino-pyrido[1,2,3-d,e][1,3,4]benzoxadiazinderivate der allgemeinen Formel (I)

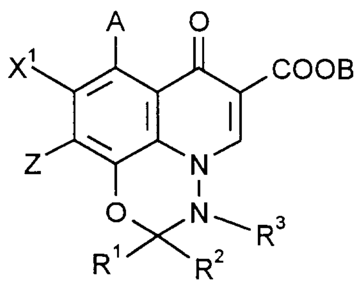

(I),

in welcher

- A für Amino oder Alkylamino steht,
- $R^1$ für Wasserstoff oder gegebenenfalls durch Hydroxy oder Halogen substituiertes $C_1$-$C_3$-Alkyl steht,
- $R^2$ unabhängig von $R^1$ für Wasserstoff oder Methyl steht,
- $R^3$ für Wasserstoff oder $C_1$-$C_4$-Alkyl steht,
- B für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- $X^1$ für Wasserstoff oder Halogen steht,
- Z für Reste der Strukturen

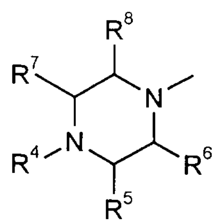
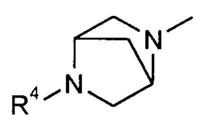
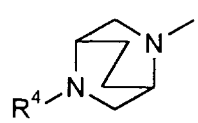
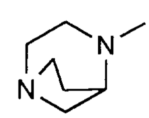

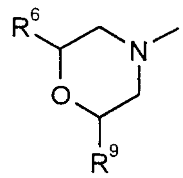
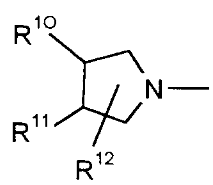
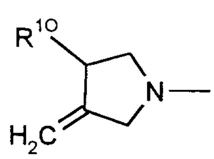
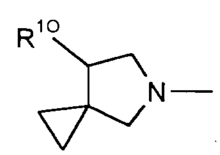

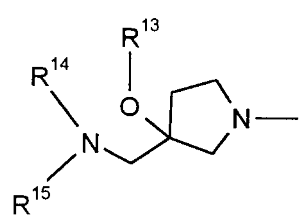
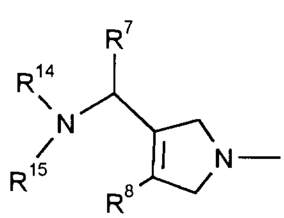
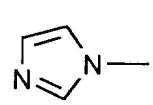
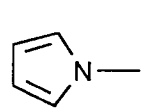

steht, worin

- $R^4$ für Wasserstoff, gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,
- $R^5$ für Wasserstoff, Methyl, Phenyl, Thienyl oder Pyridyl steht,
- $R^6$ für Wasserstoff oder $C_{1-4}$-Alkyl steht,

$R^7$ für Wasserstoff oder $C_{1-4}$-Alkyl steht,
$R^8$ für Wasserstoff oder $C_{1-4}$-Alkyl steht,
$R^9$ für Wasserstoff Methyl oder $-CH_2-NR^7R^8$ steht,
$R^{10}$ für Wasserstoff $C_{1-4}$-Alkyl, Amino, gegebenenfalls durch Hydroxy substituiertes Alkyl- oder Dialkylamino mit 1 oder 2 C-Atomen im Alkylteil, Amino-$C_{1-4}$-alkyl, $C_{1-4}$-Alkylamino-$C_{1-4}$-alkyl oder 1-Imidazolyl steht,
$R^{11}$ für Wasserstoff Hydroxy, Methoxy, Methylthio, Halogen, Methyl, Hydroxymethyl steht,
$R^{12}$ für Wasserstoff oder Methyl steht,
$R^{13}$ für Wasserstoff oder $C_{1-4}$-Alkyl steht,
$R^{14}$ für Wasserstoff oder $C_{1-4}$-Alkyl steht und
$R^{15}$ für Wasserstoff oder $C_{1-4}$-Alkyl steht,

in Form von Racematen oder als enantiomerenreine Verbindungen, ihre pharmazeutisch verwendbaren Hydrate und Säureadditionssalze sowie ihre Salze mit Basen.

**2.** Verfahren zur Herstellung der Verbindungen der Formel (I)

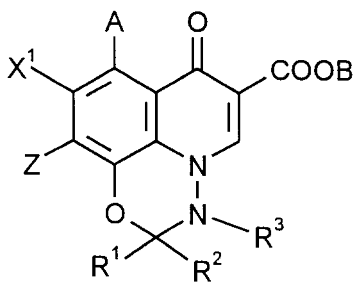

(I),

in welcher

A für Amino oder Alkylamino steht,
$R^1$ für Wasserstoff oder gegebenenfalls durch Hydroxy oder Halogen substituiertes $C_1$-$C_3$-Alkyl steht,
$R^2$ unabhängig von $R^1$ für Wasserstoff oder Methyl steht,
$R^3$ für Wasserstoff oder $C_1$-$C_4$-Alkyl steht,
B für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
$X^1$ für Wasserstoff oder Halogen steht,
Z für Reste der Strukturen

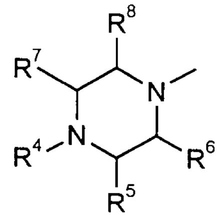 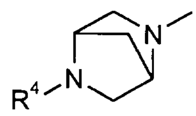 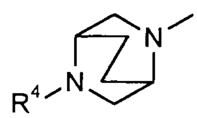 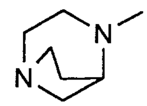

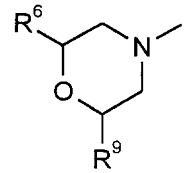 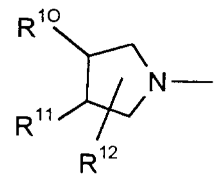 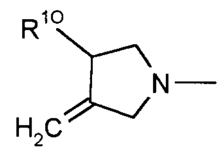 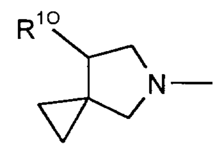

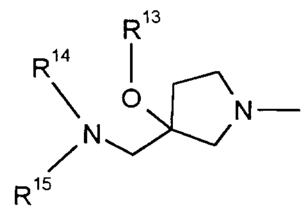 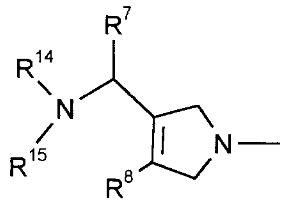 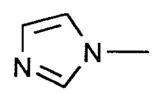 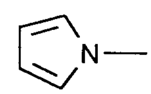

steht, worin

- $R^4$ für Wasserstoff, gegebenenfalls durch Hydroxy oder Methoxy substituiertes geradkettiges oder verzweigtes $C_1$-$C_4$-Alkyl, Cyclopropyl, Acyl mit 1 bis 3 C-Atomen steht,
- $R^5$ für Wasserstoff, Methyl, Phenyl, Thienyl oder Pyridyl steht,
- $R^6$ für Wasserstoff oder $C_{1-4}$-Alkyl steht,
- $R^7$ für Wasserstoff oder $C_{1-4}$-Alkyl steht,
- $R^8$ für Wasserstoff oder $C_{1-4}$-Alkyl steht,
- $R^9$ für Wasserstoff, Methyl oder $-CH_2-NR^7R^8$ steht,
- $R^{10}$ für Wasserstoff, $C_{1-4}$-Alkyl, Amino, gegebenenfalls durch Hydroxy substituiertes Alkyl- oder Dialkylamino mit 1 oder 2 C-Atomen im Alkylteil, Amino-$C_{1-4}$-alkyl, $C_{1-4}$-Alkylamino-$C_{1-4}$-alkyl oder 1-Imidazolyl steht,
- $R^{11}$ für Wasserstoff, Hydroxy, Methoxy, Methylthio, Halogen, Methyl, Hydroxymethyl steht,
- $R^{12}$ für Wasserstoff oder Methyl steht,
- $R^{13}$ für Wasserstoff oder $C_{1-4}$-Alkyl steht,
- $R^{14}$ für Wasserstoff oder $C_{1-4}$-Alkyl steht und
- $R^{15}$ für Wasserstoff oder $C_{1-4}$-Alkyl steht,

dadurch gekennzeichnet, daß man Verbindungen der Formel (II)

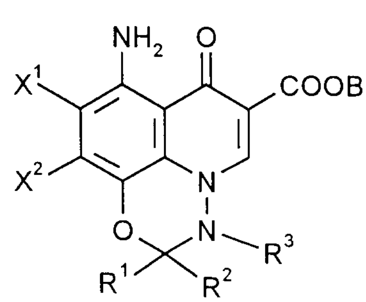 (II),

in welcher

- $R^1$, $R^2$, $R^3$, B und $X^1$ die oben angegebene Bedeutung haben und
- $X^2$ für Halogen, insbesondere Fluor oder Chlor, steht,

mit Verbindungen der Formel ((III)

Z-H (III),

in welcher

Z die oben angegebene Bedeutung hat,

gegebenenfalls in Gegenwart von Säureakzeptoren umsetzt.

**3.** Verbindungen der Formel (II)

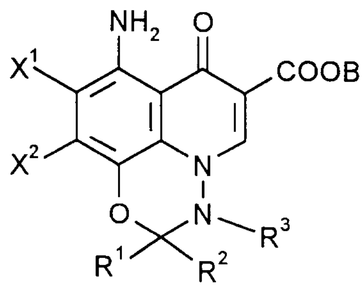

(II),

in welcher

$R^1$, $R^2$, $R^3$, B und $X^1$ die oben angegebene Bedeutung haben und

$X^2$ für Halogen, insbesondere Fluor oder Chlor, steht.

**4.** Verfahren zur Herstellung der Verbindungen der Formel (II)

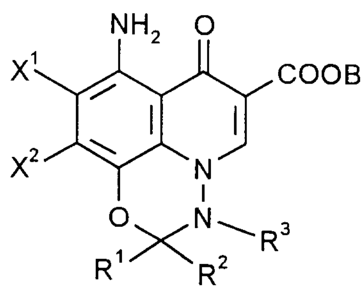

(II),

in welcher

$R^1$, $R^2$, $R^3$, B und $X^1$ die oben angegebene Bedeutung haben und

$X^2$ für Halogen, insbesondere Fluor oder Chlor, steht,

dadurch gekennzeichnet, daß man Verbindungen der Formel (IV)

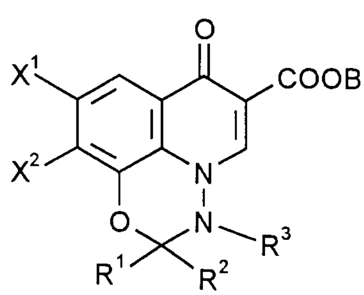

(IV),

in welcher

$R^1$, $R^2$, $R^3$, B, $X^1$ und $X^2$ die oben angegebene Bedeutung haben,

mit Nitrierungsreagenzien wie Salpetersäure und Nitraten in einem Lösungsmittel wie zum Beispiel Wasser, Schwefelsäure, Essigsäure, Acetanhydrid oder deren Gemische bei -50 °C bis 200 °C, vorzugsweise bei -20 °C bis 100 °C, umsetzt und anschließend die erhaltenen Nitroverbindungen reduziert.

**5.** Verbindungen der Formel (I) gemäß Anspruch 1, wobei

A für Amino steht,

$R^1$ für Wasserstoff oder gegebenenfalls durch OH substituiertes $C_{1-3}$-Alkyl steht,

$R^2$ unabhängig von $R^1$ für Wasserstoff oder Methyl steht,

$R^3$ für Wasserstoff, Methyl oder Ethyl steht,

B für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,

$X^1$ für Wasserstoff, Fluor oder Chlor steht,

Z für Reste der Strukturen

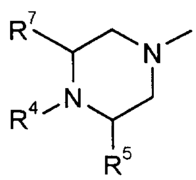
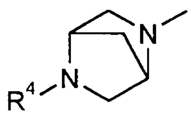
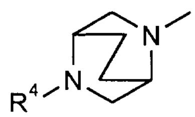
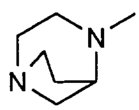

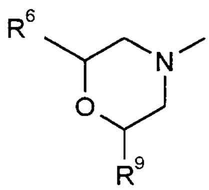
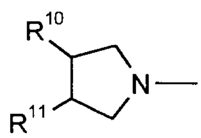
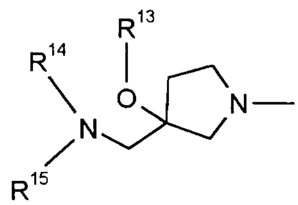
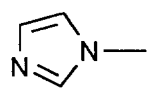

steht, worin

$R^4$ für Wasserstoff, gegebenenfalls durch Hydroxy substituiertes geradkettiges oder verzweigtes $C_1$-$C_3$-Alkyl, Oxalkyl mit 1 bis 4 C-Atomen steht,

$R^5$ für Wasserstoff, Methyl oder Phenyl steht,

$R^6$ für Wasserstoff oder Methyl steht,

$R^7$ für Wasserstoff oder Methyl steht,

$R^9$ für Wasserstoff, Methyl oder $-CH_2NH_2$ steht,

$R^{10}$ für Wasserstoff, Methyl, Amino, Methylamino, Dimethylamino, Aminomethyl, Methylaminomethyl oder Ethylaminomethyl steht,

$R^{11}$ für Wasserstoff, Hydroxy, Methyl, Fluor, Methyl oder Hydroxymethyl steht,

$R^{13}$ für Wasserstoff oder Methyl steht,

$R^{14}$ für Wasserstoff oder Methyl steht,

$R^{15}$ für Wasserstoff oder Methyl steht.

**6.** Arzneimittel enthaltend Verbindungen der Formel (I) gemäß Anspruch 1.

**7.** Verwendung von Verbindungen der Formel (I) gemäß Anspruch 1 zur Herstellung von Arzneimitteln.

**8.** Verwendung von Verbindungen der Formel (I) gemäß Anspruch 1 in antibakteriellen Mitteln.

Europäisches Patentamt

# EUROPÄISCHER RECHERCHENBERICHT

Nummer der Anmeldung
EP 95 10 6401

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | EP-A-0 259 804 (F. HOFFMANN - LA ROCHE)<br>* Ansprüche 1,40,41 * | 1,7,8 | C07D498/06<br>A61K31/535<br>//(C07D498/06, 273:00,221:00) |
| Y | JOURNAL OF ORGANIC CHEMISTRY,<br>Bd.57, Nr.2, 1992<br>Seiten 744 - 746<br>S. L. DAX ET AL 'Quinolone antibacterials: A hydroxymethylation - intramolecular cyclization route to pyrido(3,2,1-ij)-1,3,4-benzoxadiazines'<br>* das ganze Dokument * | 1,7,8 | |
| Y | JOURNAL OF MEDICINAL CHEMISTRY.,<br>Bd.33, Nr.8, 1990, WASHINGTON US<br>Seiten 2270 - 2275<br>T. P. CULBERTSON ET AL 'Quinolone antibacterial agents substituted at the 7-position with spiroamines. Synthesis and structure-activity relationships'<br>* Seite 2270; Beispiel 21q; Tabelle I * | 1,7,8 | |
| | | | RECHERCHIERTE SACHGEBIETE (Int.Cl.6) |
| Y | JOURNAL OF MEDICINAL CHEMISTRY.,<br>Bd.31, Nr.3, 1988, WASHINGTON US<br>Seiten 503 - 506<br>J. M. DOMAGALA ET AL '7-Substituted 5-amino-1cyclopropyl-6,8-difluoro-1,4-dihydro-4-oxo-3-quinolinecarboxylic acids: Synthesis and biological activity of a new class of quinolone antibacterials'<br>* Seite 503; Beispiel 2b * | 1,7,8 | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 14. Juli 1995 | Voyiazoglou, D |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)